# EUROPEAN PATENT APPLICATION

(11) **EP 3 272 341 A1**
(43) Date of publication of application: **24.01.2018**
(21) Application number: 17000525.0
(22) Date of filing: 30.03.2017
(51) Int. Cl.: A61K 9/48

(54) **ENTERIC ELASTIC FILMS FOR THE PRODUCTION OF CAPSULES**

(30) Priority: 30.03.2016 PL 41668116
(71) Applicant: Gdanski Uniwersytet Medyczny, 80-210 Gdansk (PL)
(72) Inventor: Maciejewski, Bartosz, 09-200 Sierpc (PL); Sznitowska, Malgorzata, 80-772 Gdansk (PL)
(74) Representative: Matyka, Malgorzata

(57) **Abstract**

The invention concerns an aqueous composition for the production of enteric films, containing at least one gelling polymer, enteric polymer, and water. The invention also concerns the method of producing the aqueous composition defined above, where the aqueous composition for the production of enteric films is prepared at the temperature of 60-100°C, preferably 80°C. In addition, the invention concerns the use of the enteric films containing carrageenan in the production of capsules, preferably soft capsules. -

## Description

The invention concerns enteric gelatine films for the production of capsules which enable attaining modified (delayed) release of the active substances contained in the capsule filling.

Under the current state of the art in the field of capsules, capsule shell can be made of gelatine and other materials, such as: carrageenans, alginates, starch derivatives, cellulose derivatives, (hypromellose), or gluco- and galactomannans. None of the materials used, however, demonstrates resistance to the low pH value of the stomach environment (*is gastro-resistant*). Some attempts have been made to solve the problem by e.g. making polymer gelatine - chitosan mixtures, or adding tannin, ferulic acid, gellan, and kappa-carrageenan to the composition. The above solutions, however, have proved futile in terms of the attainment of a form resistant to low pH values and cannot be used in practice in the production of soft capsules.

Soft capsules differ from the hard ones in the technology of their production, their mechanical properties, composition of the shell and the range of application. The shell of the hard (two-part) capsules is made by dipping the mould pins in a solution of film-forming polymer, followed by drying. Hard capsules are usually filled with powders, granulates, pellets, or mini-tablets, but it is also possible to fill them with non-aqueous fluids. Soft capsules have gelatine shell which is produced and filled in a single process, most often based on gelatine film formation. The ribbon/film made of the film-forming gelatine solution is formed on matrices (drums). The small container formed after partial fusion of two such films is filled with fluid or semi-solid content and sealed up.

To make the capsules gastro-resistant, i.e. to ensure they will disintegrate only when they reach the intestines, filled capsules are coated with an appropriate polymer which is insoluble in the stomach. The method is highly problematic, especially in the case of soft capsules. It would be most advantageous to make enteric capsules of a material demonstrating 'gastro-resistant' properties. Today, the method consisting in the modification of capsule shell based on crosslinking of gelatine so as to 'harden' it is losing in popularity because of poor solubility of the thus-obtained forms irrespective of the pH value of the environment, the potential presence of traces of the substances (often toxic, e.g. formaldehyde) used in the crosslinking process in the ready capsule, or the uncontrollability of the process related strictly to its nature.

Attempts have been taken to develop a capsule shell material which would include film-forming polymers soluble at pH higher than that of gastric fluid, such as methacrylic acid copolymers (Eudragit L, S), cellulose acetate phthalate, or hypromellose phthalate.

Patent descriptions US8685445 and US2014/0348879 disclose the composition of a soft capsule shell material made of a film-forming mass comprising a gelling polymer (e.g. gelatine), acid-insoluble polymer, and an alkaline aqueous solvent.

Disclosed in patent description WO2013/164122 is the composition of a film-forming mixture devoid of any alkalizing substances, designed for the production of capsules (5-50% of enteric polymer in the form of dispersion, at least one excipient in the amount of 0.5-20%, and water). The composition according to the invention is suitable for use in the production of hard capsules. It prevents, however, making a film which would set forming a continuous film structure, elasticity of which would be sufficient for the purposes of producing soft capsules in the typical production process, i.e. the continuous process of capsule formation and filling.

Patent description WO2013/164121 discloses capsule composition comprising hypromellose acetate succinate (HPMCAS), with an alkalizing substance added to partially neutralize groups of the succinic acid in the polymer molecule. The proposed compositions enable producing hard capsules by the dipping method, they cannot be, however, used in the process of soft capsule production.

The purpose of this invention is to develop the materials intended for gelatine capsule shells which would enable delayed release (in the intestines) of the substances from both hard and soft capsules. Such materials could be obtained by modifying the polymers currently used in capsule production. The main purpose is to develop the capsule shell compositions which would not contain any alkalizing substances and use them in the technology of producing both hard capsules by the dipping method, and soft capsules by the rotary die method.

The gelatine films used in soft capsule production must demonstrate specific mechanical properties, i.e. they must be tear-resistant and elastic while in production, thanks to which wet gelatine film can be fed onto the matrix drums, shaped, and filled when already in the capsule form. In addition, the material must be adhesive to enable sealing together the sides of the capsules after filling to close them up.

The invention concerns aqueous composition for the production of enteric films, containing at least one gelling polymer, enteric polymer, and water.

Aqueous composition where gelatine is used as the gelling polymer in the amount of 17% - 37%.

Aqueous composition where enteric polymer is selected from among derivatives of the methacrylic acid, cellulose acetate phthalate, hypromellose phthalate, or hypromellose acetate succinate.

Aqueous composition where the enteric polymer is present in the amount ranging from 14% to 34%, preferably amounting to 17% based on dry mass.

Aqueous composition where the enteric polymer is used in the form of dispersion and is not converted to soluble form.

Aqueous composition where the gelatine-enteric polymer weight ratio ranges from 3:2 to 4:1, preferably 3:1.

Aqueous composition which contains 30-60% of water, preferably 33%.

Aqueous composition containing no hydroxides, salts, amines, or other additives which increase the pH value.

Aqueous composition which additionally contains a plasticizer.

Aqueous composition where the plasticizer is any of the following: glycerol, propylene glycol, or macrogol in the amount ranging from 0.1% to 30%, preferably staying between 10% and 21%.

Aqueous composition which demonstrates properties which enable its use in the capsule forming process.

The invention concerns the method of producing the aqueous composition defined above, where the aqueous composition for the production of enteric film is prepared at the temperature of 60-100°C, preferably at 80°C.

The method, where to prepare the enteric film, aqueous dispersion of cellulose acetate phthalate is combined with a gelling polymer at any temperature not higher than 65° C.

The method in which the composition sets as the temperature decreases, thus enabling the formation of enteric film.

The invention also concerns an enteric film which after the drying process contains 0.1-20% of water.

Enteric films which do not disintegrate in acidic environment where the pH value ranges from 1.2 to 4.5.

Enteric films which do not disintegrate in acidic pepsin solutions.

Enteric films which disintegrate in any environment at pH ≥5.5.

Enteric films which demonstrate properties making them suitable for use in the capsule formation process.

Enteric films which demonstrate improved elasticity and tear resistance thanks to the presence of an additional gelling polymer in their composition.

Enteric films in which carrageenan is used as the additional gelling polymer.

Enteric films in which carrageenan is present in the amount of 0.2% to 1.0%.

Enteric films, elasticity of which measured as elongation at break at thickness of ca. 0.4 mm exceeds 150% and preferably stands above 260%.

Enteric films for which tear resistance measured as force is more than 0.1 N, preferably more than 1 N, at thickness of ca. 0.4 mm.

Enteric films, the resistance of which to 0.1 M HCl measured as mass undissolved after 30 min. grows and is no lower than 50%.

Use of the enteric films described above in the production of capsules, preferably soft capsules.

The composition of the capsule shell according to the invention is advantageous compared to the similar previously described gelatine films modified with enteric polymers in that it does not contain any alkalizing substances. In effect, the shell is resistant to disintegration in a broader range of acidic pH values (i.e. between pH 1 and pH 4.5), does not disintegrate when exposed to surfactants and enzymes, and demonstrates higher elasticity and mechanical resistance, which makes it suitable for use also in the technology of producing soft (elastic) capsules.

### Figure description:

**Fig. 1** - shows the disintegration time of the films according to example 12 and example 5.
**Fig.2** - shows the disintegration time of the film according to example 5 in compounded media. 1) SGF pH 1.2, 2) SGF +pepsin (pH 1.2), 3) FaSSGF (pH 1.6), 4) pH 3.0 citrate buffer, 5) pH 3.0 phosphate buffer, 6) pH 4.5 phosphate buffer, 7) pH 4.5 acetate buffer, 8) FeSSIF (pH 5.0), 9) pH 5.5 phosphate buffer, 10) pH 6.0 phosphate buffer, 11) FaSSIF (pH 6.5), 12) pH 6.8 phosphate buffer.
**Fig.3** - depicts film disintegration time, test duration: 180 min.
**Fig.4** - shows the effects of iota-carrageenan added to the composition on elasticity of the films produced (light bars represent the films which were setting for 5 min. at the temperature of 8°C, dark bars represent the films after drying).
**Fig.5** - presents the force needed to disrupt the structure continuity of a set film depending on the gelatine source and the quantity of carrageenan added.
**Fig.6** - shows changes in film mass over time following immersion in 0.1 M HCl, depending on the quantity of carrageenan added (0.5-1.5% based on dry mass).

The invention is illustrated with the following examples which do not limit the invention:

### Description of the tests to determine the disintegration time, mechanical resistance, and viscosity of the solution.

Disintegration test - fragments of **dried** films (1x1cm, water content 1-4%) were placed in conical flasks, each containing 50 ml of, respectively: simulated gastric fluid FP X (pH 1.2), citrate buffer pH 3.0, phosphate buffers pH 3.0, 4.5, 5.5, 6.0 and 6.8. The flasks were shaken for 3h at the temperature of 37°C at the rate of 150 rpm. Similarly, the selected films were tested for 2h in the following fluids: simulated gastric fluid pH 1.2 added with: pepsin (FP X, Ph. Eur.), FaSSGF (simulated gastric fluid 'fasted state', pH 1.6, with an addition of bile salts and lecithin), acetate buffer pH 4.5, FeSSIF (simulated intestinal fluid 'fed state' with an addition of bile salts and phospholipids, pH 5.0), FaSSIF (simulated intestinal fluid 'fasted state' with an addition of bile salts and phospholipids, pH 6.5). The samples were checked for disintegration every 5 minutes.

Mechanical properties test- fragments of **non-dried** films sized 1x5cm were placed in the setup for uniaxial tensile tests of the TA.XT Plus texture analyser (Stable Micro Systems, Godalming, UK). The tests measured the force needed to tear the film samples and their elongation at which the break occurred.

Film-forming mass viscosity tests - samples of the aqueous enteric dispersion were placed on the thermostated plate of a Haake Viscotester 550 viscometer (Haake, Karlsruhe, Germany) at the temperature of 80°C, whereupon viscosity in the plate-cone system was measured (PK 1 1°, gap 0.05mm, rotor speed: 10 rps).

In the examples, the following column titles are used: '% - solution' denoting the percentage content of the components in the aqueous composition; '% - dry film denoting the percentage content of the components in dried film which simulates the shell of prepared capsules. Used as plasticizers were glycerol, propylene glycol or macrogol 400. It is permissible for dry films comprising a plasticizer to contain a minor quantity of water the presence of which results from the drying conditions and the nature of the material (<10%). In the films which do not contain a plasticising agent permitted is water content of at last ca. 15% to prevent film brittleness.

The enteric polymers, cellulose acetate phthalate and methacrylates were used in the form of commercially-available aqueous dispersions. Aquacoat CPD (produced by FMC Biopolymer, Philadelphia, USA), is a 30% aqueous dispersion containing: cellulose acetate phthalate (CAP), poloxamer and water. Eudragit L30 D55 (Evonik, Essen, Germany) is a 30% aqueous dispersion containing: methacrylic acid - ethyl acrylate copolymer, ca. 0.7% of sodium dodecyl sulphate, ca. 2.3 % of polysorbate 80 and water.

### Example 1. (hard capsule shell)

| **Component** | **%-solution** | **%** - **dry film** |
|---|---|---|
| Gelatine | 17% | 41% |
| AquacoatCPD | 37% | 28% |
| Macrogol 400 | 13% | 31% |
| Water | 34% | |

### Example 2.(hard capsule shell)

| **Component** | **%-solution** | **%** - **dry film** |
|---|---|---|
| Gelatine | 21% | 52% |
| AquacoatCPD | 23% | 17% |
| Glycerol | 13% | 31% |
| Water | 44% | |

### Example 3.(hard capsule shell)

| **Component** | **%-solution** | **%** - **dry film** |
|---|---|---|
| Gelatine | 22% | 55% |
| AquacoatCPD | 18% | 14% |
| Glycerol | 13% | 31% |
| Water | 47% | |

### Example 4.(soft capsule shell)

| **Component** | **%-solution** | **%** - **dry film** |
|---|---|---|
| Gelatine | 21.7% | 57.9% |
| AquacoatCPD | 18.3% | 14.6% |
| Propylene glycol | 10.0% | 26.7% |
| Carrageenan | 0.3% | 0.8% |
| Water | 49.7% | |

### Example 5.(soft capsule shell)

| **Component** | **%-solution** | **% - dry film** |
|---|---|---|
| Gelatine | 20.0% | 50.0% |
| AquacoatCPD | 22.9% | 17.2% |
| Glycerol | 12.5% | 31.3% |
| Carrageenan | 0.6% | 1.5% |
| Water | 44.0% | |

### Example 6.(soft capsule shell)

| **Component** | **%-solution** | **% - dry film** |
|---|---|---|
| Gelatine | 21.4% | 53.5% |
| AquacoatCPD | 18.3% | 13.8% |
| Glycerol | 12.5% | 31.2% |
| Carrageenan | 0.6% | 1.5% |
| Water | 47.2% | |

### Example 7.(soft capsule shell)

| **Component** | **%-solution** | **% - dry film** |
|---|---|---|
| Gelatine | 19.6% | 49.1% |
| AquacoatCPD | 22.9% | 17.2% |
| Glycerol | 12.5% | 31.2% |
| Carrageenan | 1.0% | 2.5% |
| Water | 44.0% | |

### The method of preparing the solutions and films according to the composition given in examples 1-7:

1. Adding the specified amounts of the plasticizer and water into the vessel.
2. Mixing them and heating the mixture to 60°C (ca. 15 min)
3. Adding carrageenan (if applicable), stirring for 10 min.
4. Adding gelatine (stirring for about 20 min)
5. Adding 'enteric' polymer (pre-heated to the temperature of 60°C, stirring for 15 minutes)
6. Increasing the temperature to 80°C and stirring until homogenous
7. Casting the mixture on a flat surface
8. Dryingat room temperature

### Properties of the films obtained (non-dried)

| **Film composition** | **Visual assessment** | **Ease of separation from the substrate (plate)** | **Elongation at break (EAB [%])** | **Tear resistance (TR[N])** | **Film-forming solution viscosity (assessment/ measurement)** |
|---|---|---|---|---|---|
| Example 1 | Homogenous, cohesive | Rather hard to separate | Not tested | Not tested | Low / not tested |
| Example 2 | Homogenous, cohesive | Rather easy to separate | 135% | 0.17 N | Low / not tested |
| Example 3 | Homogenous, cohesive | Rather easy to separate | 138% | 0.30 N | Low / not tested |
| Example 4 | Homogenous, cohesive | Easy to separate | 199% | 0.32 N | Medium/not tested |
| Example 5 | Homogenous, cohesive | Easy to separate | 238% | 0.3 1 N | Medium /2.9 Pa×s· |
| Example 6 | Homogenous, cohesive | Easy to separate | 190% | 0.48 N | Medium / not tested |
| Example 7 | Homogenous, cohesive | Easy to separate | 234 % | 0.39 N | Medium/ not tested |

### Example 8.(hard or soft capsule shell)

| **Component** | **%-solution** | **%** - **dry film** |
|---|---|---|
| Gelatine | 35% | 51% |
| Aquacoat CPD | 38% | 17% |
| Glycerol | 21% | 31% |
| Water | 6% | |

### Example 9.(hard or soft capsule shell)

| **Component** | **%-solution** | **%** - **dry film** |
|---|---|---|
| Gelatine | 36.8% | 54.9% |
| AquacoatCPD | 30.7% | 13.8% |
| Glycerol | 21.0% | 31.3% |
| Water | 11.5% | |

### Example 10.(soft capsule shell)

| **Component** | **%-solution** | **%** - **dry film** |
|---|---|---|
| Gelatine | 33.5% | 50.0% |
| AquacoatCPD | 38.3% | 17.2% |
| Glycerol | 21.0% | 31.3% |
| Carrageenan | 1.0% | 1.5% |
| Water | 6.2% | |

### Example 11. (hard or soft capsule shell)

| **Component** | **%-solution** | **%** - **dry film** |
|---|---|---|
| Gelatine | 20.0% | 50.0% |
| Hypromellose | | |
| phthalate | 6.9% | 17.3% |
| Glycerol | 12.5% | 31.3% |
| Carrageenan | 0.6% | 1.5% |
| Water | 60.0% | |

### The method of preparing the solutions and films according to the composition given in examples 8-11

1. Adding the specified amounts of enteric polymer, glycerol and water into the vessel.
2. Mixing them and heating the mixture to ca. 50°C
3. Adding carrageenan (if applicable), stirring until dissolution
4. Adding gelatine (increasing the temperature to 80°C)
5. Stirring until homogenous
6. Casting the mixture on a flat surface
7. Drying at room temperature

### Properties of the films obtained (non-dried)

| **Film composition** | **Visual assessment** | **Ease of separation from the substrate (plate)** | **Elongation at break (EAB [%])** | **Tear resistance (TR[N])** | **Film-forming solution viscosity (assessment/ measurement)** |
|---|---|---|---|---|---|
| Example 8 | Homogenous, cohesive | Very easy to separate | 267% | 1.4 N | High / 21.9 Pa×s |
| Example 9 | Homogenous, cohesive | Very easy to separate | Not tested | Not tested | High / Not tested |
| Example 10 | Homogenous, cohesive | Very easy to separate | 264% | 1.3 N | Very high / 50.9 Pa×s |
| Example 11 | Cohesive, solid particles visible | Easy to separate | Not tested | Not tested | Low / Not tested |

### Example 12.(soft capsule shell)

| **Component** | **%-solution** | **%** - **dry film** |
|---|---|---|
| Gelatine | 20.0% | 50.0% |
| AquacoatCPD | 22.9% | 17.2% |
| Glycerol | 12.5% | 31.3% |
| Carrageenan | 0.6% | 1.5% |
| Water | 44.0% | |

### The method of preparing the films according to the composition given in example 12

1. Adding the specified amounts of glycerol and water into the vessel
2. Mixing them and heatting the mixture to 65°C (ca. 15 min)
3. Adding carrageenan (stirring for 10 min)
4. Adding gelatine (stirring for ca. 20 min)
5. Adding enteric polymer (pre-heated to 65°C, stirring for 15 min)
6. Stirring at 65°C until homogenous.
7. Casting the mixture on a flat surface.
8. Drying at room temperature.

### Comparison of the disintegration times of films prepared at65°C and 80°C

Fig.1

### Comparative example 13.(with an alkalizing agent added)

| **Component** | **%-solution** | **% - dry film** |
|---|---|---|
| Gelatine | 19.3% | 48.4% |
| Aquacoat CPD | 21.5% | 16.2% |
| Glycerol | 12.3% | 30.8% |
| NaOH | 1.8% | 4.6% |
| Water | 45.1% | |

### Disintegration times of films according to Example 13 in different fluids, as compared to Examples 1 and 2:

| **Medium** | **Example 2** | **Example 1** | **Comparative example 13** |
|---|---|---|---|
| SGF | No disintegration | No disintegration | <5 min |
| pH 3.0 citrate buffer | No disintegration | No disintegration | <5 min |
| pH 3.0 phosphate buffer | No disintegration | No disintegration | <5 min |
| pH 4.5 phosphate buffer | No disintegration | No disintegration | <5 min |
| pH 5.5 phosphate buffer | 15 - 30 min | 5 - 15 min | <5 min |
| pH 6.0 phosphate buffer | 5 - 15 min | 5 - 15 min | <5 min |
| pH 6.8 phosphate buffer | 5 - 10 min | 5 - 10 min | <5 min |

### Disintegration time of the film according to example 5 in compounded media:

Fig.2

### Example 14.(hard capsule shell)

| **Component** | **%-solution** | **%** - **dry film** |
|---|---|---|
| Gelatine | 13.1% | 33.8% |
| Eudragit L30- | | |
| D55 | 43.8% | 33.9% |
| Glycerol | 12.5% | 32.3% |
| Water | 30.6% | |

### Example 15.(hard capsule shell)

| **Component** | **%-solution** | **% - dry film** |
|---|---|---|
| Gelatine | 20.8% | 53.7% |
| Eudragit L30-D55 | 18.0% | 14.0% |
| Glycerol | 12.5% | 32.3% |
| Water | 48.7% | |

### Example 16.(hard capsule shell)

| **Component** | **%-solution** | **%** - **dry film** |
|---|---|---|
| Gelatine | 15.8% | 40.8% |
| Eudragit L30-D55 | 34.7% | 26.9% |
| Glycerol | 12.5% | 32.3% |
| Water | 37% | |

### The method of preparing the films according to the composition given in examples 14-16

1. Adding the specified amounts of glycerol and water into the vessel
2. Mixing them and heating the mixture to 65°C (ca. 15 min)
3. Adding gelatine (stirring for about 20 min)
4. Adding enteric polymer (pre-heated to 65°C, stirring for 15 min)
5. Stirring in 65°C until homogenous.
6. Casting the mixture on a flat surface
7. Dryingat room temperature.

### Example 17. (hard capsule shell)

| **Component** | **%-solution** | **% - dry film** |
|---|---|---|
| Gelatine | 20.6% | 60.0% |
| Aquacoat CPD | 22.9% | 20.0% |
| Water | 56.5% | 20.0% |

### The method of preparing the solutions and films according to example 17

1. Adding the specified amounts of enteric polymer and water into the vessel
2. Mixing them and heating the mixture to ca. 50°C
3. Adding gelatine (and increasing the temperature to 100°C)
4. Stirring until homogenous
5. Casting the mixture on a flat surface
6. Dryingat room temperature.

### Example 18. (hard or soft capsule shell)

| **Component** | **%-solution** | **%** - **dry film** |
|---|---|---|
| Gelatine | 20.0% | 50.0% |
| Hypromellose acetate succinate | 6.9% | 17.3% |
| Glycerol | 12.5% | 31.3% |
| Carrageenan | 0.6% | 1.5% |
| Water | 60.0% | |

### The method of preparing the solution and film according to example 18

1. Adding the specified amounts of enteric polymer, glycerol and water into the flask.
2. Mixing them and heating the mixture to ca. 50°C
3. Adding carrageenan (if applicable), stirring until dissolution
4. Adding gelatine (and increasing the temperature to 80°C)
5. Stirring until homogenous
6. Casting the mixture on a flat surface
7. Drying at room temperature.

### Disintegration times of films according to examples 14-16:

Fig.3

### Mechanical properties of the films, depending on the type of gelatine used and on the presence of carrageenan in the composition:

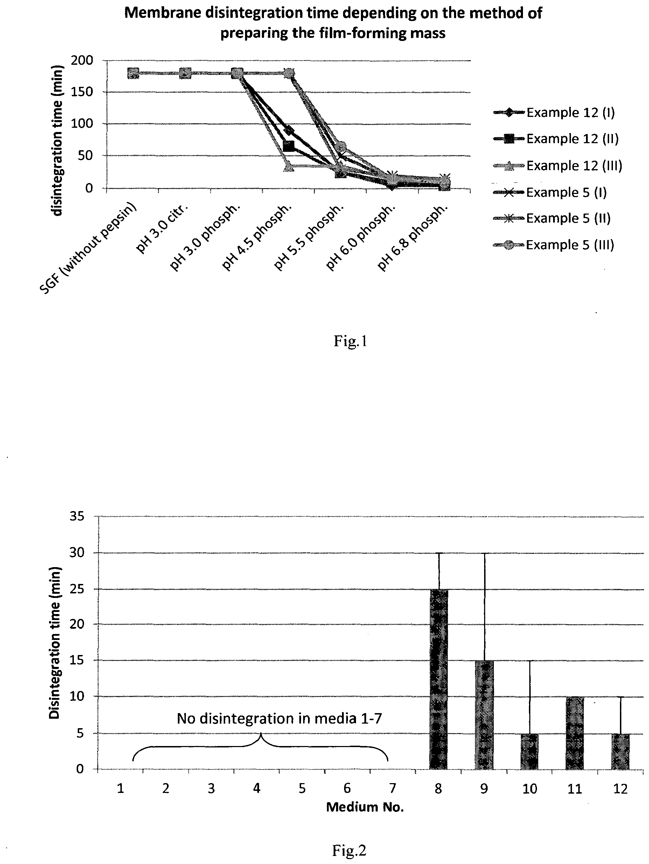

The findings indicate that 0.6% of carrageenan added to the composition increased substantially the value of the elongation at break parameter (by more than 100% when pork or fish gelatine was used) as compared to the compositions not containing carrageenan. The results of the elasticity tests (expressed as the Elongation at Break parameter [EAB]) are shown in fig. 1. It is also evident that the kind and source of the gelatine used is also important alongside the addition of carrageenan. Thanks to improved film elasticity the described formulation can be used in the process of producing soft capsules by the rotary die method.

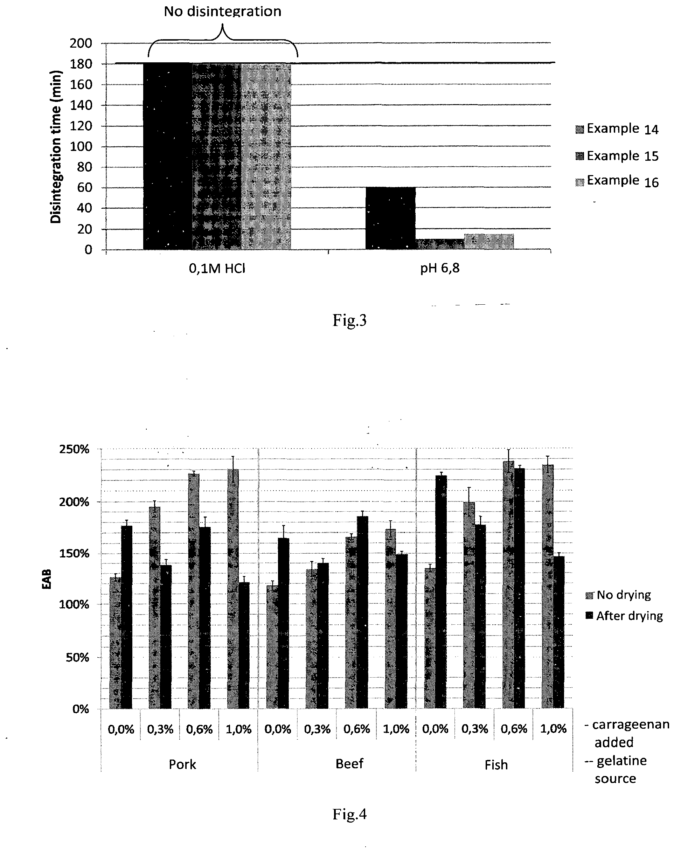

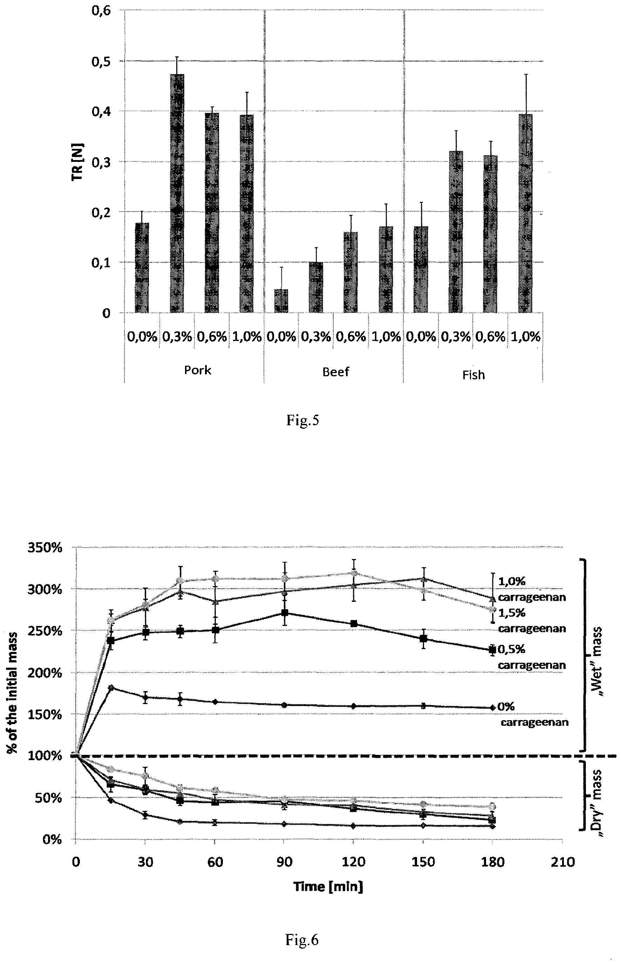

The force needed to break film continuity was also measured (Tear Resistance parameter - TR [N]). Subject to testing were filmsset at room temperature, non-dried (crucial for the soft capsule production process, fig. 2). The results reveal that presence of carrageenan in the composition improves mechanical resistance of the films. Fig.4 and Fig.5

### Changes in film mass after immersion in 0.1 M HCl, depending on presence of carrageenan in the composition (0.5-1.5% based on dry mass)

Mass increase during immersion in 0.1 M hydrochloric acid (37°C) was measured. The measurements were conducted for dried films containing no carrageenan, or containing 0.2%, 0.4% and 0.6% of carrageenan (0.5%, 1.0% and 1.5% when based on dry mass). The results indicate the mass increases substantially in compositions containing carrageenan along with the swelling process as compared to films containing no carrageenan.

Film samples immersed in acid were investigated for changes of dry mass over the immersion time. The results indicate that addition of carrageenan to the composition resulted in substantially lower loss of mass in the films as compared to the films containing no carrageenan. After 30 min the mass of carrageenan-free films dropped to ca. 30% of the initial mass, while in those with 0.5 - 1.5% of carrageenan the mass reduced to 50-75% of the initial mass. The effect depends on carrageenan concentration in the composition and may increase the barrier properties of films when immersed in a fluid of low pH value. - Fig. 6

### Literature:

1. Arvanitoyannis I.S., Nakayama A., Aiba S. "Chitosan and gelatin based edible films: state diagrams, mechanical and permeation properties", Carbohydrate Polymers 37 (1998) 371-382
2. Benameur H., Hutchinson K., "Aqueous dispersions of controlled release polymers and shells and capsules thereof", Zg 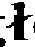 oszenie patentowe nr WO 2013/164122, 2013.
3. Brown M.D. "Improvements in or relating to encapsulation", Patent nr WO9735537, 1996.
4. Cade, D.N., Straub H., "Aqueous dispersions of hydroxypropyl methylcellulose acetate succinate (hpmcas)", Zg 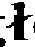 oszenie patentowe nr WO 2013/164121, 2013.
5. Cao N., Fu Y., He J. "Mechanical properties of gelatin films cross-linked, respectively, by ferulic acid and tannin acid", Food Hydrocolloids 21 (2007) 575-584
6. Draper P.R. i in. "Film forming compositions comprising modified starches and iota-carrageenan and methods for manufacturing soft capsules using same", Patent nr WO0103677, 1999.
7. Felton LA i in. Physical and enteric properties of soft gelatin capsules coated with Eutragit L30 D-55. International Journal of Pharmaceutics 113 (1995) 17-24.
8. Gennadios A. "Non-gelatin substitutes for oral delivery capsules, their composition and process of manufacture", Patent nr US 6214376 B1, 1998.
9. Hassan E.M., Fatmi A.A., Chidambaram N., "Enteric composition for the manufacture of soft capsule wall", Patent nr US 8685445, 2014.
10. Hassan E.M., Fatmi A.A., Chidambaram N., "Enteric soft capsule", Zg 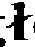 oszenie patentowe nr US 2014/0348879, 2014.
11. Jones R.T. "Gelatin: Manufacture and physio-chemical properties" w: Podczeck F., Jones B.E. "Pharmaceutical Capsules" Pharmaceutical Press 2004, s. 23-60
12. Karim A. A., Bhat,R."Gelatin alternatives for the food industry: recent development, challenges and prospects". Trends in Food Science & Technology, 19 (2008) 644-656.
13. Menard R. i in. "Sposób wytwarzania wyrobu kszta 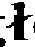 towego zawieraj 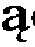 cego skrobi 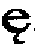 , kompozycja zawieraj 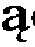 ca skrobi 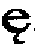 , wyrób kszta 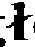 towy zawieraj 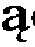 cy skrobi 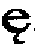 oraz urz 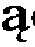 dzenie do wytwarzania mi 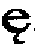 kkiej kapsu 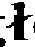 ki zawieraj 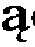 cej skrobi 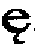 ", Patent nr PL204120, 2000
14. Pranoto, Y., Lee, C. M., Park, H. J. "Characterizations of fish gelatin films added with gellan and k-carrageenan." LWTFood Science and Technology 40(2007) 766-774
15. Reich G. "Formulation and physical properties of soft capsules", w: Podczeck F., Jones B.E. "Pharmaceutical Capsules" Pharmaceutical Press 2004, s. 201-212
16. Winston P.E., Jr.; Miskiel F.J.; Valli R.C. "Composition and process for gelatin-free soft capsules", Patent nr US 5342626, 1994.
17. Enteric Aqueous Coating [http://www.fmcbiopolymer.com/Portals/bio/content/Docs/AquaCoat%20CPD%207706%20. pd f]- dost 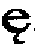 p 25.03.2016
18. Maciejewski B., Weitschies W., Schneider F., Sznitowska M."Gastroresistant gelatin films prepared by addition of cellulose acetate phthalate", Pharmazie 72 (2017) [IN PRESS]

## Claims

1. Aqueous composition for the production of enteric films, containing at least one gelling polymer, enteric polymer, and water.

2. Aqueous composition according to Claim 1, **wherein**gelatine is used as the gelling polymer in the amount of 17% - 37%.

3. Aqueous composition according to Claim 1, **wherein** enteric polymer is selected from among derivatives of the methacrylic acid, cellulose acetate phthalate, hypromellose phthalate, or hypromellose acetate succinate.

4. Aqueous composition according to Claim 1, **wherein** the enteric polymer is present in the proportion ranging from 14% to 34%, preferably amounting to 17% based on dry mass.

5. Aqueous composition according to Claim 1, **wherein** the enteric polymer is used in the form of dispersion and is not converted to soluble form.

6. Aqueous composition according to Claim 1., **wherein** the gelatine-enteric polymer weight ratio ranges from 3:2 to 4:1, preferably 3:1.

7. Aqueous composition according to Claim 1, **wherein** it contains 30-60% of water, preferably 33%.

8. Aqueous composition according to Claim 1, **wherein** it contains no hydroxides, salts, amines, or other additives which increase the pH value.

9. Aqueous composition according to Claim 1, **wherein**itadditionally contains a plasticizer.

10. Aqueous composition according to Claim 9, **wherein** the plasticizer is any of the following: glycerol, propylene glycol, or macrogol in the amount ranging from 0.1 % to 30%, preferably staying between 10% and 21%.

11. Aqueous composition according to Claim 1, **wherein** it demonstrates properties that enable its use in the capsule forming process.

12. The method of producing the aqueous composition defined in Claim 1, **wherein** the aqueous composition for the production of enteric film is prepared at the temperature of 60-100°C, preferably at 80°C.

13. The method according to Claim 12, **wherein** to prepare the enteric film an aqueous dispersion of cellulose acetate phthalate is combined with a gelling polymer at any temperature not higher than 65° C.

14. The method according to Claim 12, **wherein** the composition congeals as the temperature decreases, thus enabling the formation forming of enteric film.

15. The enteric film according to Claim 12, **wherein** after the drying process it contains 0.1-20% of water.

16. The enteric films according to Claim 15, **wherein** they do not disintegrate in acidic environment where the pH value ranges from 1.2 to 4.5.

17. The enteric films according to Claim 15, **wherein** they do not disintegrate in acidic pepsin solutions.

18. The enteric films according to Claim 15, **wherein** they disintegrate in any environment at pH ≥5.5

19. The enteric films according to Claim 15, **wherein** they demonstrate properties making them suitable for use in the capsule formation process.

20. The enteric films according to Claim 15, **wherein** they demonstrate improved elasticity and tear resistance thanks to the presence of an additionalgelling polymer in their composition.

21. The enteric films according to Claim 20, **wherein**carrageenan is used as the additionalgelling polymer.

22. The enteric films according to Claim 20, **wherein** their elasticity measured as elongation at break at thickness of ca. 0.4 mm exceeds 150% and preferably stands above 260%

23. The enteric films according to Claim 21, **wherein** carrageenan is present in their composition in the amount of 0.2% to 1.0%.

24. The enteric films according to Claim 21, **wherein**tear resistance measured as force is more than 0.1 N, preferably more than 1 N, at thickness of ca. 0.4 mm.

25. The enteric films according to Claim 21, **wherein** their resistance to 0.1 M HCl measured as mass undissolved after 30 min. grows and is no lower than 50%.

26. Use of the enteric films according to Claims 20-25 in the production of capsules, preferably soft capsules.
